# EUROPEAN PATENT APPLICATION

(11) **EP 1 057 801 A2**
(43) Date of publication of application: **06.12.2000**
(21) Application number: 00110987.5
(22) Date of filing: 29.05.2000
(51) Int. Cl.: C07B 39/00, C07C 17/14, C07C 67/307, C07C 69/76

(54) **Process for benzylic bromination**

(30) Priority: 01.06.1999 US 323336
(71) Applicant: ALBEMARLE CORPORATION, Baton Rouge, LA 70801-1765 (US)
(72) Inventor: Mortensen, Max K., Louisiana 70816 (US); Elnagar, Hassan Y., Louisiana 70810 (US); Roy, Ranjit K., Louisiana 70816 (US); Herndon, Robert C., Jr., LA 70816 (US); Allen, Robert H., LA 70808 (US); Caillet, David A., LA 70816 (US)
(74) Representative: UEXKÜLL & STOLBERG

(57) **Abstract**

A process for benzylic bromination of a wide variety of benzylic compounds is described. Bromine is used as the bromination agent. Moderate temperatures are employed and the process can be used to produce in relatively pure form either mono- or dibrominated benzylic bromination products.

## Description

### BACKGROUND

The formation of benzyl and benzal bromide compounds from benzylic compounds is an important step in the synthesis of a wide variety of organic products. Such compounds, formed by the mono- and dibromination, respectively, of a benzylic carbon atom, are used in the production of a wide variety of pharmaceutical products, as well as other compounds. For example, the benzyl bromide of ethyl 4-methyl benzoate is an intermediate in the synthesis of Eprosartan, a promising new antihypertensive drug. The benzal bromides are extremely useful as well in that they can be converted into aldehydes in a process which replaces the two benzylic bromine atoms with a single oxygen atom.

Many methods of benzylic bromination with bromine are known. Bromine is often used as the bromination reagent. Ultraviolet radiation and radical initiators such as AIBN have been used to facilitate the addition of bromine to the benzylic carbon atom.

It has been known for some time that benzylic bromination of some types of benzylic compounds can be accomplished in the absence of actinic radiation, catalyst or reaction initiator by thermal means. Such thermal benzylic bromination processes can be convenient in that they do not require light sources or additional catalyst/initiator removal steps.

Heretofore, benzylic bromination processes have been characterized by a lack of flexibility as to which bromination product is produced. Thus, even if a benzylic bromination process has been able to rid the reaction of hydrogen bromide, such a process typically has not readily permitted the switching of production from one bromination product to the other.

Furthermore, many benzylic bromination processes lack the ability to produce a solution of even a moderately pure benzyl or benzal bromide product. Instead, they are constrained to produce mixtures in which both products are present in appreciable amounts. Thus, separation steps must undertaken if a pure product is desired.

A process for benzylic bromination with bromine which is capable of producing relatively pure benzyl or benzal bromide product, or mixtures of both containing prescribed proportions of each, as desired, would represent a significant advance in the state of the art.

### SUMMARY OF THE INVENTION

It has been discovered that when the bromination reaction is conducted at a range of moderate temperatures, and the brominating agent is bromine gas which is blended with the benzylic substrate in such a way as to suppress localized accumulation of bromine, the production of benzal bromide can be reduced to impurity amounts. Thus, a first embodiment of this invention is a process for producing a benzyl bromide which process comprises:
a) contacting gaseous bromine with a reaction mixture having an organic, liquid phase initially comprising an aromatic compound having a ring substituent which has at least two hydrogen atoms in the alpha-position, the total amount of bromine relative to said aromatic compound being in the range of from about 0.2 to about 1.2 mole of bromine per mole of said aromatic compound, more preferably in the range of from about 0.4 to about 0.6 mole of bromine per mole of said aromatic compound;
b) thoroughly dispersing the gaseous bromine into said liquid phase reaction mixture such that localized bromine accumulation therein is suppressed; and
c) having the temperature of said liquid organic phase in the range of about 100°C to about 170°C sufficient to effect benzylic bromination of said ring substituent.

Furthermore, it has been found that when benzylic bromination is conducted in accordance with the aforementioned method, production of the benzal product from the benzyl product can be conveniently effected by simply continuing to supply the bromine to the reaction while the reaction temperature is maintained in the aforementioned range of moderate temperatures until a product mixture is formed in which the benzal bromide is present in the desired proportions. Thus, a second preferred embodiment of the process of this invention is a process for producing a benzal bromide which process comprises:
a) contacting gaseous bromine with a reaction mixture having an organic, liquid phase initially comprising an aromatic compound having a ring substituent which has at least two hydrogen atoms in the alpha-position, preferably a methyl group, the total amount of bromine relative to said aromatic compound being in the range of from about 1.5 to about 2.5 moles of bromine per mole of said aromatic compound, more preferably in the range of from about 2.0 to about 2.2 moles of bromine per mole of said aromatic compound;
b) thoroughly dispersing the gaseous bromine into said liquid phase reaction mixture such that localized bromine accumulation therein is suppressed; and
c) having the temperature in the range of about 100°C to about 170°C sufficient to effect dibromination of said substituent.

A particularly advantageous mode of contacting the bromine with the aromatic compound used in the process of this invention is to introduce it into the reaction mixture via subsurface feeding. Thus, a third embodiment of the inventive process disclosed herein is a process for producing a benzyl bromide, which process comprises:
a) feeding gaseous bromine directly into and below the surface of a reaction mixture having an organic, liquid phase and comprising an aromatic compound having a ring substituent which has at least two hydrogen atoms in the alpha-position, the total amount of bromine relative to said aromatic compound being in the range of from about 0.2 to about 1.2 moles of bromine per mole of said aromatic compound, more preferably in the range of from about 0.4 to about 0.6 mole of bromine per mole of said aromatic compound;
b) thoroughly dispersing said feed of gaseous bromine within said liquid organic phase such that localized bromine accumulation therein is suppressed; and
c) having the temperature in the range of about 100°C to about 170°C sufficient to effect benzylic bromination of said ring substituent.

As aromatic compounds such as esters can react with hydrogen bromide, a byproduct produced in the bromination reaction described herein, it is within the scope of this invention to implement a method for the removal of hydrogen bromide when using such aromatic compounds in the process of this invention.

The inclusion of an aqueous phase in the reaction mixture of a) in the embodiments above can effectively dissolve hydrogen bromide produced in the bromination reaction, removing it from the aqueous layer, and in doing so, prevent the degradation of the aromatic bromination substrate. Thus, other embodiments of the process of this invention as in the three embodiments above, wherein said reaction mixture includes water in an amount sufficient to effect at least partial removal of byproduct HBr.

The mixing of bromine with one of more inert gases, such as nitrogen, prior to the contacting of the bromine with the reaction mixture of a) in the embodiments above, is yet another efficient mode for the removal of the hydrogen bromide from the reaction mixture. Additionally, such diluents can aid in the suppression of localized bromine accumulation in the reaction mixture. Accordingly, other embodiments of the inventive process described herein are as in the embodiments above, wherein said gaseous bromine is diluted with at least one inert gas.

Yet another effective mode of hydrogen bromide removal is the utilization of an organic solvent, which, when included in the reaction mixture of a), will allow the reaction mixture to reflux at a temperature in the range of from about 100°C to about 170°C. Such refluxing transfers the hydrogen bromide to the headspace above the reaction mixture surface. Thus, further embodiments of the process of this invention are as in the first three embodiments above, wherein said reaction mixture further comprises an inert liquid solvent, and wherein the gaseous coproduct HBr is removed from the reaction mixture at a rate sufficient to maintain the concentration of HBr in the organic liquid phase of said reaction mixture below about 5 wt% based on the weight of said organic liquid phase.

The above and other embodiments will be apparent from the ensuing description and appended claims.

### FURTHER DESCRIPTION OF THE INVENTION

A wide variety of benzylic compounds can be used as the substrate in the conduction of the process of this invention. Suitable are various aromatic ring-containing compounds bearing one benzylic carbon atom, which itself bears at least two, and preferably three hydrogen atoms, such as, 9-ethylanthracene, 2-methylanthracene, 2-bromoethylbenzene, 2-methylstyrene, 2-methyl-5-nitrobenzonitrile, 2-methylbenzonitrile, 2-chloro-4-methylbenzoic acid, 4,6-hydroxy-orthotoluic acid, the ethyl ester of 2,4-dihydroxy-6-methylbenzoic acid, 3-ethylbenzoic acid, 2-methylbenzoic acid, 2-amino-4'-methylbenzophenone, 2-hydroxy-5-methyl-benzophenone, 2-methylbenzophenone, 4-methyl-2-nitrobenzophenone, 4-methyl-2'-nitrobenzophenone and pcresol. More suitable are benzylic compounds which contain one benzylic carbon atom, as above, but additionally, which benzylic carbon atom is borne on an aromatic ring structure comprised of a single ring. Substitutions on this ring are permissible. Most preferred are ethyl 4-methylbenzoate, ethyl 4-ethyl benzoate, toluene, parabromotoluene, and parafluorotoluene, and 4-bromo-2-fluorotoluene.

If it is desired to prepare a benzal bromide compound, it is within the scope of this invention to carry out the bromination process set forth herein on a benzyl bromide compound. The benzyl bromide derivatives of the compounds disclosed above are suitable substrates. As above, suitable are the benzyl bromides of benzylic compounds which contain a single benzylic carbon atom. More suitable are compounds containing a single benzylic carbon atom, which is borne on an aromatic ring structure comprised of a single ring which is, optionally, substituted. Most suitable are the benzyl bromides of methyl toluate, ethyl toluate, parabromotoluene, 4-bromo-2-fluorotoluene and parafluorotoluene, with the first three being exceptionally desirable.

It is to be noted that certain benzylic substrates, especially those bearing ether linkages or other ring activating substituents, may initially undergo nuclear bromination instead of benzylic bromination. However, once ring bromination has taken place, benzylic bromination according to the process of this invention can begin to take place.

Some of the benzylic substrates above are available though Aldrich or other chemical supply houses. The simpler starting compounds, such as methyl toluate, para-cresol and parabromotoluene, as well as some of the more familiar multi-ring compounds, such as 2-methylanthracene, are readily obtainable.

The temperature at which the bromination is conducted is a feature of the invention described herein. It is preferable to utilize a temperature in the range of from about 100°C to about 170°C. Many compounds which form a relatively large proportion of benzyl bromide product at temperatures in the lower portion of the above range when utilized in the process of this invention. Such compounds will typically form mixtures which are predominantly the benzal bromide product when they are subjected to the process of this invention at temperatures which are in the middle and upper portions of the above range (e.g., in the range of from about 125°C to about 170°C).

It is not necessary to adhere to anhydrous reaction conditions when conducting the process of this invention. Typically, the presence of water will not materially impair the effectiveness of the benzylic bromination reaction. It is therefore not necessary to use anhydrous bromine in conducting the process of this invention. Likewise, the inert gas need not be rigorously dried.

The feeding of the bromine gas into the reaction mixture at a point underneath the surface of the mixture can aid in the suppression of localized accumulation of bromine. It is preferable to finely disperse the bromine in the liquid organic phase of the reaction mixture as such dispersion further reduces the likelihood of pockets of increased bromine concentration.

Preferably, the gaseous bromine is fed to the reaction mixture either continuously or substantially continuously so as not to unduly prolong the operation and thus impair productivity as a function of time. However, it is permissible to interrupt the feed without adverse consequences with respect to the selectivity of the reaction.

As noted above, some compounds which can be used in the process of this invention are sensitive to hydrogen bromide in appreciable amounts. Benzylic esters are particularly prone to degradation through cleavage when hydrogen bromide is present. Thus, the elimination of hydrogen bromide from the reaction mixture can be beneficial.

The utilization of an aqueous phase can aid in the removal of hydrogen bromide. When using an aqueous phase, it is preferable to use a mole ratio of water to benzylic starting compound in the range of from 2 to about 20 moles of water per mole of benzylic compound. As agitation of the reaction mixture is required, it can be expected that the aqueous phase of the reaction mixture will be dispersed in the liquid organic phase of the reaction mixture during the bromination reaction, effecting efficient hydrogen bromide removal.

Another method for reducing the presence of hydrogen bromide in the reaction mixture is the dilution of the bromine with one or more inert gases prior to the addition of the bromine to the reaction mixture. As the inert gas leaves the reaction mixture, it removes substantial amounts of hydrogen bromide. The mixing of bromine with one or more inert gases gives additional benefits in that the inert gas serves to reduce the formation of the benzal bromide product to levels below those observed in the absence of the gas (as illustrated in the comparative example hereinafter).

Many different inert gases can be used as bromine diluents. If desired, two or more such gases can be used. Suitable examples include noble gases, such as helium, neon, argon, krypton and xenon. Other examples include carbon dioxide and nitrogen. Nitrogen is preferable.

It is desirable that the volume ratio of inert gas to bromine be in the range of from about 10 to about 500 mL of inert gas per mL of bromine. More desirable is an inert gas/bromine volume ratio in the range of from about 10 to about 300 mL of inert gas per mole of bromine. A volume ratio in the range of from about 50 to about 150 is most desirable.

When conducting the process of this invention, the inert gas can be mixed with the bromine at a convenient temperature, such as room temperature, using a "T"- tube, a static mixer, or the like, before introducing the bromine/inert gas mixture into the reaction zone. The bromine can be evaporated before or during the time it is being mixed with the inert gas.

The inclusion of an ancillary solvent is yet another way to effect the removal of hydrogen bromide. It is particularly suitable for closed systems which do not allow for the removal of the diluent gases mentioned above. By refluxing the reaction mixture, hydrogen bromide is effectively translocated from the reaction mixture to the headspace above the reaction mixture. A variety of different solvents can be used, provided that they are inert to bromination, they do not substantially interfere with the bromination reaction, and the reaction mixture, upon inclusion of the solvent, refluxes at a temperature in the range of from about 100°C to about 170°C. Preferable are halogen-containing solvents, such as ethylene dichloride, carbon tetrachloride and bromobenzene. Most preferable is chlorobenzene. In the case of benzylic compounds which are susceptible to interaction with hydrogen bromide, such as esters, it is advantageous to employ a solvent in which hydrogen bromide has a low solubility, preferably about 1 gram of hydrogen bromide or less per 100 grams of solvent, at a temperature of 25°C.

Typically, solvents are used in amounts such that the weight ratio of benzylic starting compound to solvent is in the range of from about 0.2 to about 1.5 grams of benzylic starting compound per gram of solvent. A weight ratio of from about 0.8 to about 1.0 moles of benzylic compound per mole of solvent is more preferable.

Bromine must be used in amounts great enough to effect the production of the desired bromination product. Typically, if a product mixture in which the benzyl bromide product predominates is desired, it is preferable to employ bromine in amounts such that the mole ratio of bromine to benzylic compound is in the range of from about 0.2 to about 1.2 moles of bromine per mole of benzylic compound. More preferable is a mole ratio of from about 0.3 to about 0.7 moles of bromine per mole of benzylic compound. Most preferable when forming a product rich in the benzyl bromide product is a ratio of bromine to benzylic compound which is in the range of from about 0.4 to about 0.6 moles of bromine per mole of benzylic compound.

If a mixture which predominates in the benzal bromide product is desired, it is preferable to have a ratio of bromine to benzylic compound ratio in the range of from about 1.5 to about 2.5 moles of bromine per mole of benzylic compound. A mole ratio in the range of from about 1.8 to about 2.3 moles of bromine per mole of benzylic compound is more preferable. Most preferable is a mole ratio in the range of from about 2.0 to about 2.2 moles of bromine per mole of benzylic compound.

It is notable that the ratio of benzyl bromide to benzal bromide can exhibit a dependence upon several variables. One such variable is the level of substrate conversion to which the bromination reaction is run. In general, low conversion of substrate to benzyl and benzal bromides is associated with a higher ratio, whereas high conversion tends to give a lower ratio. A feature of this invention is the high benzyl/benzal bromide ratio which can be obtained even at high levels of substrate conversion.

Another variable which can affect the ratio of benzyl bromide to benzal bromide is the bromine addition rate. In order to attain an optimum value for the product ratio, it is preferable to contact the bromine with the benzylic compound at a rate in the range of from about 0.05 to about 3.0 grams of bromine per minute per gram mole of benzylic compound. Most preferable is a rate in the range of from about 0.1 to about 0.5 grams of bromine per minute per gram mole of benzylic compound. If a mixture which predominates in the benzyl bromide product is desired, a gradual or incremental reduction in the rate of bromine addition during the conversion of the benzylic substrate into benzyl bromide can be beneficial.

The degree to which the reaction mixture is agitated is an additional variable which can have a bearing on the selectivity of the reaction for the benzyl bromide product. Agitation means such as a shaker, or rocking autoclave can be insufficient to effect the mixing required to eliminate local accumulation of the bromine in the reaction mixture. Thus, mechanical stirring is preferred because of its ability to quickly and finely disperse the bromine/inert gas mixture in the benzylic compound. A rate of agitation which is in the range of from about 200 to about 1000 RPM is desirable.

The degree to which each of the above factors affects the benzyl bromide to benzal bromide ratio can vary from with different compounds. It is thus advisable to do a trial run to determine their effect for a particular compound.

As a slight negative pressure aids in the separation of the hydrogen bromide byproduct from the reaction mixture, the pressure under which the reaction is conducted is preferably in the range of from 0.8 to about 1 atmosphere. Most preferably, the pressure is about 1 atmosphere.

Variations of the inventive process described herein are possible. For example, instead of conducting the process in the manner of the examples, the bromine and the benzylic compound can be continuously combined, with agitation, and flowed through a heated reaction zone for a time suitable to produce the desired amount of benzyl and/or benzal product.

The reaction time depends on the degree of benzylic compound conversion desired. In addition, different benzylic compounds are benzylically brominated at different rates. The conversion of a benzylic compound to its benzyl bromide in about 70% yield generally takes a time in the range of from about 2 hours to about 15 hours, and the conversion of a benzylic compound to its corresponding benzal bromide generally takes a time in the range of from about 10 hours to about 25 hours.

The benzyl and benzal bromides which are produced in the process of this invention can be separated and purified with conventional methods such as extraction, distillation, and recrystallization. The separation and purification methods used can be chosen with respect to the properties of the benzyl/benzal bromides produced.

Examples 1-4 below illustrate thermal benzylic brominations conducted pursuant to this invention.

### EXAMPLE 1

### Benzylic Bromination of p-Bromotoluene (p-BT)

Charged to 3.0 kg (17.54 mol) of p-BT of a purity of >99.6%, gaseous bromine mixed with nitrogen was continuously fed at the rate of 1.00 mL/min). During the feeding the temperature was maintained at 110°C, and the reaction mixture was stirred at 600 RPM. Periodically during the feeding samples of the reaction mixture were taken and analyzed by GC and NMR for the ratio of the p-bromobenzyl bromide to p-bromobenzalbromide. Table 1 summarizes the results.

**TABLE 1**

| Br₂ added (g) | No. of moles of Br₂ | Mole % of Br₂ | Benzyl/Benzal |
|---|---|---|---|
| 616 | 3.85 | 22.0 | 62.7 |
| 1168 | 7.30 | 41.7 | 25.0 |
| 1783 | 11.15 | 63.6 | 13.1 |
| 2387 | 14.93 | 85.2 | 8.3 |

### EXAMPLE 2

### Benzylic Bromination of p-Bromotoluene (p-BT)

A second run was performed as in Example 1 except that 1.0 kg (5.85 mol) of p-bromotoluene was used. In addition, the run was to a lesser degree of conversion of p-BT. In all other respects, the reaction conditions were the same. The results are summarized in Table 2.

**TABLE 2**

| Br₂ added (g) | No. of moles of Br₂ | Mole % of Br₂ | Benzyl/Benzal |
|---|---|---|---|
| 122 | 0.76 | 13.1 | 109 |
| 244 | 1.53 | 26.1 | 57.3 |
| 367 | 2.30 | 39.3 | 34.2 |
| 490 | 3.06 | 52.4 | 23.9 |

### COMPARATIVE EXAMPLE

The procedure of Examples 1 and 2 was repeated using a liquid bromine feed instead of a gaseous bromine/nitrogen mixture. The results are summarized in Table 3.

**TABLE 3**

| Br₂ added (g) | No. of moles of Br₂ | Mole % of Br₂ | Benzyl/Benzal |
|---|---|---|---|
| 121 | 0.76 | 12.9 | 112 |
| 246 | 1.54 | 26.3 | 66.2 |
| 372 | 2.33 | 39.7 | 33.5 |
| 495 | 3.10 | 52.8 | 19.7 |

### EXAMPLE 3

### Benzylic Bromination of Ethyl 4-Methylbenzoate (EMB)

An ethyl 4-methylbenzoate solution (1134 g in chlorobenzene) was charged into a 2-Liter round-bottomed flask equipped with a mechanical stirrer (300 RPM stirring rate) and a cooling condenser, and heated. The heating was set at 140°C, and a scrubber was connected to the condenser. Liquid bromine (275 g, 1.72 mol or approximately 59 mol%, Aldrich, 99.5+, A.C.S. reagent) was fed via Viton tubing and the bromine feed rate was adjusted to 1.00 mL per minute for the first 30 mol% bromine, then to 0.50 mL per minute for the rest of the addition at 140°C. Heating and stirring continued for a few minutes after all bromine was added. The hot mixture was then allowed to cool to obtain 1183 grams crude reaction mixture (some materials were used for routine analysis). The GC weight% analysis of the crude reaction mixture indicated 17.2% of starting EMB (theoretical: 203.6 g) and 28.0% of ethyl 4-(bromomethyl) benzoate (EBMB) (theoretical: 331.5 g). Distillation under reduced pressure removed first the solvent, chlorobenzene, then the unreacted starting material (EMB, total of 192.6 g, approximately 95% of theoretical, at 19 Torr, 120°C) as a colorless oil. Next, the desired EBMB was recovered (283.3 g, approximately 86% of theoretical, 18 Torr, 174°C)as a low melting solid. The ratio of ethyl 4-(bromomethyl) benzoate to ethyl 4-(dibromomethyl) benzoate was determined by gas chromatography to be 14.0 GC percent.

### EXAMPLE 4

### Benzylic Bromination of Methyl 4-Methylbenzoate

A methyl 4-methylbenzoate solution (110 g, 0.73 mol) in 250 mL round-bottomed flask was heated by a heating mantle connected to a temperature controller, with stirring (approximately 300 RPM) to temperatures between 136 and 140°C. Liquid bromine (84.4 g, 0.53 mol, approximately 72 mol%, Aldrich 99.5+%, A.C.S. reagent) was fed via Viton tubing using a Cole-Parmer Computerized Drive with Easy-Load pump head. The bromine feed rate was adjusted to 0.50 mL per minute throughout the 50 minute addition time. Heating and stirring continued for a few minutes after all bromine was added. The reaction mixture was allowed to cool. The orange mixture was transferred to a separatory funnel and washed with water(250 mL) to produce a colorless organic phase. The bottom layer (organic) was separated and concentrated under reduced pressure (rotary evaporator) to remove most of the chlorobenzene. The residual oily mixture was allowed to stand overnight. An impure crystalline solid was obtained. The crude solid was dissolved in a 20% aqueous ethanol solution (450 mL) to produce 50.5 g (42% based on added bromine) of highly (approximately 99%) material. No attempt was made to optimize the recovered yield. The ratio of methyl 4-(bromomethyl) benzoate to methyl 4-(dibromomethyl) benzoate was determined by gas chromatography to be 14.0 GC percent.

### EXAMPLE 5

### Benzylic Bromination of 4-Bromo-2-fluorotoluene (BFT)

In a 0.5 kg thermal benzylic bromination reaction, bromine (mixed with nitrogen, 0.65 mL/min) was fed (at 132 C, 1000 RPM) and the consumption of the starting material, and the production of the mono- and dibrominated material was monitored. In this case the reaction in question is the bromination of 4-bromo-2-fluoro toluene to selectively produce 4-bromo-2-fluorobenzyl bromide and 4-bromo-2-fluorobenzal bromide. The reaction was carried out in a 500 mL kettle reactor using an overhead high power stirring system. The bromine was premixed with nitrogen and fed through a heat exchanger so as to completely vaporize the bromine, and the reaction operated at atmospheric pressure. The bromine was fed at a rate of approximately 0.49 gram/min. The off gas was collected and analyzed in a gas scrubber for HBr. The bromine feed rate was extended beyond stoichiometric monobromination in order to study the ratio of monobrominated to dibrominated product. The reaction mixture was periodically sampled and analyzed by gas chromatography. Table 4 summarizes the data obtained, and the data are expressed in terms of area %.

**TABLE 4**

| **Bromine Feed** %Stoichiometric Bromine Feed | **BFT:** %4-bromo-2-fluoro toluene in reaction crude | **BFBB:** %4-bromo-2-fluoro benzyl bromide in reaction crude | **BFBDB:** %4-bromo-2-fluoro benzal bromide in reaction crude | **BFBB/BFBDB** Ratio |
|---|---|---|---|---|
| 0.00 | 99.47 | 0.00 | 0.00 | 0.00 |
| 24.30 | 77.38 | 21.36 | 0.44 | 48.55 |
| 52.20 | 52.98 | 43.77 | 2.43 | 18.01 |
| 75.10 | 34.09 | 59.21 | 5.76 | 10.28 |
| 101.53 | 20.71 | 67.69 | 10.60 | 6.39 |
| 107.79 | 16.35 | 69.96 | 12.75 | 5.49 |
| 150.00 | 0.36 | 51.24 | 47.50 | 1.08 |
| 170.00 | 0.00 | 34.42 | 58.48 | 0.59 |
| 190.00 | 0.00 | 18.45 | 75.97 | 0.24 |
| 200.00 | 0.00 | 8.73 | 77.12 | 0.11 |
| 210.00 | 0.00 | 5.96 | 86.36 | 0.07 |

A second reaction, under the same conditions, was run and the results are shown in Table 5. In this case the samples were analyzed for the actual wt% within the reaction crude. This was done so that an accurate evaluation of the conversion, selectivity, and % yield could be obtained. The reaction selectivity for the runs summarized in Tables 4 and 5 was high in both cases. Selective production of either the mono- or dibrominated material is thus made possible.

**TABLE 5**

| **Bromine Feed Time:** Min | **BFT:** wt%4-bromo-2-fluoro toluene in reaction crude | **BFBB:** wt%4-bromo-2-fluoro benzyl bromide in reaction crude | **BFBDB:** wt%4-bromo-2-fluoro benzal bromide in reaction crude | **BFBB/BFBDB** Ratio |
|---|---|---|---|---|
| 0.00 | 92.9 | 0.1 | 0.1 | 0 |
| 960 | 10.7 | 52.5 | 17 | 3.09 |
| 1024 | 0.2 | 41.4 | 55.6 | 0.74 |
| 1060 | 0.1 | 25.3 | 62.1 | 0.41 |
| 1107 | 0.1 | 14 | 87.3 | 0.16 |
| 1140 | 0.1 | 3.4 | 84.9 | 0.04 |

It is to be understood that the reactants and components referred to by chemical name or by formula anywhere in the specification or claims hereof, whether referred to in the singular or plural, are identified as they exist prior to coming into contact with another substance referred to by chemical name or chemical type (e.g., another reactant, a solvent, or etc.). It matters not what preliminary chemical changes, transformations and/or reactions, if any, take place in the resulting mixture or solution or reaction medium as such changes, transformations and/or reactions are the natural result of bringing the specified reactants and/or components together under the conditions called for pursuant to this disclosure. Thus the reactants and components are identified as ingredients to be brought together in connection with performing a desired chemical reaction or in forming a mixture to be used in conducting a desired reaction. Accordingly, even though the claims hereinafter may refer to substances, components and/or ingredients in the present tense ("comprises," "is," etc.), the reference is to the substance, component or ingredient just as it existed at the time just before it was first contacted, blended or mixed with one or more other substances, components and/or ingredients in accordance with the present disclosure. Thus the fact that a substance, component or ingredient may have lost its original identity through a chemical reaction or transformation through the course of contacting, blending or mixing operations, if conducted in accordance with this disclosure and with the application of common sense and the ordinary skill of a chemist, is thus wholly immaterial for an accurate understanding and appreciation of the true meaning and substance of this disclosure and the claims thereof.

This invention is susceptible to considerable variation in its practice. Therefore the foregoing description is not intended to limit, and should not be construed as limiting, the invention to the particular exemplifications presented hereinabove. Rather, what is intended to be covered is as set forth in the ensuing claims and the equivalents thereof permitted as a matter of law.

## Claims

1. A process for producing a benzyl bromide which process comprises:
a) contacting gaseous bromine with a reaction mixture having an organic, liquid phase initially comprising an aromatic compound having a ring substituent which has at least two hydrogen atoms in the alpha-position, the total amount of bromine relative to said aromatic compound being in the range of from about 0.2 to about 1.2 moles of bromine per mole of said aromatic compound;
b) thoroughly dispersing the gaseous bromine into said liquid phase, such that localized bromine accumulation therein is suppressed; and
c) having the temperature of said liquid phase in the range of about 100°C to about 170°C sufficient to effect benzylic bromination of said ring substituent.

2. A process of Claim 1 wherein said aromatic compound is an ester, and wherein HBr byproduct is removed from said reaction mixture.

3. A process of Claim 2 wherein said reaction mixture includes water in an amount sufficient to effect at least partial removal of byproduct HBr.

4. A process of Claim 1 wherein said gaseous bromine is diluted with at least one inert gas.

5. A process of Claim 4 wherein at least a portion of the inert gas removes from said reaction mixture at least a portion of the HBr byproduct.

6. A process of Claim 5 wherein said reaction mixture includes water in an amount sufficient to effect at least partial removal of byproduct HBr.

7. A process of any of Claims 1-6 wherein said aromatic compound is p-bromotoluene, p-fluorotoluene, or 4-bromo-2-fluorotoluene.

8. A process of Claims 2, 3, 5, or 6 wherein said aromatic compound is methyl 4-methylbenzoate or ethyl 4-methylbenzoate.

9. A process for producing a benzal bromide which process comprises:
a) contacting gaseous bromine with a reaction mixture having an organic, liquid phase initially comprising an aromatic compound having a ring substituent which has at least two hydrogen atoms in the alpha-position, the total amount of bromine relative to said aromatic compound being in the range of from about 1.5 to about 2.5 moles of bromine per mole of said aromatic compound;
b) thoroughly dispersing the gaseous bromine into said liquid phase reaction mixture such that localized bromine accumulation therein is suppressed; and
c) having the temperature in the range of about 100°C to about 170°C sufficient to effect dibromination of said substituent.

10. A process of Claim 9 wherein said aromatic compound is an ester, and wherein HBr byproduct is removed from said reaction mixture.

11. A process of Claim 10 wherein said reaction mixture includes water in an amount sufficient to effect at least partial removal of byproduct HBr.

12. A process of Claim 9 wherein said gaseous bromine is diluted with at least one inert gas.

13. A process of Claim 12 wherein at least a portion of the inert gas removes from said reaction mixture at least a portion of the HBr byproduct.

14. A process of Claim 13 wherein said reaction mixture includes water in an amount sufficient to effect at least partial removal of byproduct HBr.

15. A process of any of Claims 9-14 wherein said aromatic compound is p-bromotoluene, p-fluorotoluene, or 4-bromo-2-fluorotoluene.

16. A process of Claims 10, 11, 13, or 14 wherein said aromatic compound is methyl 4-methylbenzoate or ethyl 4-methylbenzoate.

17. A process for producing a benzyl bromide, which process comprises:
a) feeding gaseous bromine directly into and below the surface of a reaction mixture having an organic, liquid phase and initially comprising an aromatic compound having a ring substituent which has at least two hydrogen atoms in the alpha-position, the total amount of bromine relative to said aromatic compound being in the range of from about 0.2 to about 1.2 moles of bromine per mole of said aromatic compound;
b) thoroughly dispersing said feed of gaseous bromine within said liquid organic phase such that localized bromine accumulation therein is suppressed; and
c) having the temperature in the range of about 100°C to about 170°C sufficient to effect benzylic bromination of said ring substituent.

18. A process of Claim 17 wherein HBr byproduct is removed from said reaction mixture.

19. A process of Claim 18 wherein said reaction mixture includes water in an amount sufficient to effect at least partial removal of byproduct HBr.

20. A process of Claim 17 wherein said gaseous bromine is diluted with at least one inert gas.

21. A process of Claim 17 wherein said aromatic compound is an ester and HBr byproduct is removed from said reaction mixture.

22. A process of Claim 21 wherein said gaseous bromine is diluted with at least one inert gas, and wherein at least a portion of the inert gas removes from said reaction mixture at least a portion of the HBr byproduct.

23. A process of Claim 22 wherein said reaction mixture includes water in an amount sufficient to effect at least partial removal of byproduct HBr from said liquid organic phase.

24. A process of any of Claims 17-20 wherein said aromatic compound is p-bromotoluene, p-fluorotoluene, or 4-bromo-2-fluorotoluene.

25. A process of Claims 21, 22, or 23 wherein said aromatic compound is methyl 4-methylbenzoate or ethyl 4-methylbenzoate.

26. A process of Claim 17 wherein said aromatic compound is an ester, wherein said reaction mixture further comprises an inert liquid solvent, and wherein the gaseous coproduct HBr is removed from the reaction mixture at a rate sufficient to maintain the concentration of HBr in the organic liquid phase of said reaction mixture below about 5 wt% based on the weight of said organic liquid phase.

27. A process of Claim 17 wherein said reaction mixture is maintained at reflux during at least a substantial portion of the time the bromine is being fed.

28. A process of Claim 1 wherein the total amount of bromine relative to said aromatic compound is in the range of from about 0.4 to about 0.6 mole of bromine per mole of said aromatic compound.

29. A process of Claim 17 wherein the total amount of bromine relative to said aromatic compound is in the range of from about 0.4 to about 0.6 mole of bromine per mole of said aromatic compound.
